# EUROPEAN PATENT SPECIFICATION

(11) **EP 3 854 331 B1**
(45) Date of publication and mention of the grant of the patent: **03.01.2024**
(21) Application number: 21150044.2
(22) Date of filing: 04.01.2021
(51) Int. Cl.: A61B 17/70, A61B 17/88

(54) **MULTI-CAP REMOVING-AND-HOLDING INSTRUMENT FOR SPINAL SURGERIES**
INSTRUMENT ZUM ENTFERNEN UND HALTEN MEHRERER KAPPEN FÜR WIRBELSÄULENOPERATIONEN
INSTRUMENT DE RETRAIT ET DE MAINTIEN MULTI-CAPUCHONS POUR LES CHIRURGIES DE LA COLONNE VERTÉBRALE

(30) Priority: 21.01.2020 US 202016748081
(43) Date of publication of application: 28.07.2021
(73) Proprietor: Warsaw Orthopedic, Inc., Warsaw, IN 46582 (US)
(72) Inventor: GLASER, Adam, Germantown, Tennessee (US); BROWN, R. Quinn, Collierville, Tennessee (US); WALL, Daniel, Cordova, Tennessee (US); SIMPSON, Joshua, Collierville, Tennessee (US)
(74) Representative: Viering, Jentschura & Partner mbB Patent- und Rechtsanwälte

(56) References cited:
- US-A1- 2008 255 576
- US-A1- 2011 296 961
- US-A1- 2013 296 888
- US-B1- 6 634 261
- US-B1- 9 907 582

## Description

### FIELD

The present disclosure relates to systems for use in spinal surgeries, generally, and, more particularly to systems for removing multiple implant-assembly caps in surgery.

### BACKGROUND

Spinal pathologies and disorders such as scoliosis, kyphosis, and other curvature abnormalities, degenerative disc disease, disc herniation, osteoporosis, spondylolisthesis, stenosis, tumor and fracture may result from factors including trauma, disease and degenerative conditions caused by injury and aging. Spinal disorders typically result in symptoms including deformity, pain, nerve damage, and partial or complete loss of mobility.

Non-surgical treatments, such as medication, rehabilitation and exercise can be effective, however, may fail to relieve the symptoms associated with these disorders.

Surgical treatment of these spinal disorders includes correction, fusion, fixation, discectomy, laminectomy and implantable prosthetics.

Surgical rods are used commonly in correcting spinal abnormalities. Pedicle-screw assemblies are often used to facilitate securement of one or more spinal rods relative to the spine. Pedicle-screw assemblies include a bonescrew attached to a rod-receiving receiver. The bonescrews are attached to patient vertebrae, and the receivers receive portions of the spinal rod.

The receivers of typical pedicle-screw assemblies are in some cases angularly positionable with respect to the bonescrew to facilitate select orientation of the spinal rod with respect to the vertebrae. With the bonescrews fixed to the vertebrae, a user can in connecting the rod to the receiver persuade the spine toward a desired shape.

Percutaneous pedicle fixation is a minimally invasive surgical technique involving placing pedicle screws and spinal rods through very small skin incisions. Surgeons in some cases attach external extender instruments to heads of the pedicle screws. The extender instruments can facilitate rod reduction or maneuvering the rod into place in the heads.

There is need for spinal implant systems that enable robust rod reduction without external extender instruments, and instruments facilitating use thereof.

US 2011/296961 A1 discloses an apparatus for tightening frangible collar fasteners and retaining separated nut portions of fasteners according to the preamble of claim 1. Further apparatus for dispensing locking caps are disclosed in US 2013/296888 A1 and US 2008/255576 A1.

### SUMMARY

Systems and processes of the present disclosure relate generally to monolithic percutaneous-screw systems for use in spinal surgeries, such as minimally invasive spinal surgeries. The present invention relates to a multiple-cap removing-and-holding instrument as claimed hereafter. Preferred embodiments are set forth in the dependent claims.

Details of various aspects of the disclosure are set forth in the accompanying drawings and description below. Other features, objects, and advantages of the technology will be apparent from the description, drawings, and claims.

### BRIEF DESCRIPTION OF DRAWINGS

FIG. 1 is a perspective view of a monolithic percutaneous pedicle screw system according to an example which does not fall within the scope of the claims as such but represents disclosure helping in understanding the present invention;
FIG. 2 is a perspective view of a proximal portion of a receiver assembly connected by a first, distal, breakoff section to a distal portion of an intrinsic extender of the system of FIG. 1;
FIG. 3 is a perspective view of a guide cap connected by a second, proximal, breakoff section to a proximal portion of the intrinsic extender of the system of FIG. 1;
FIG. 4 is a side view of the parts shown in FIG. 3;
FIG. 5 is a perspective cross-section of the parts in FIG. 3;
FIG. 6 is a perspective view of the proximal portion of the intrinsic extender after the guide cap has been broken off of the extender;
FIG. 7 shows a guide-cap remover instrument according to one embodiment of the present invention, adjacent the monolithic percutaneous pedicle screw system;
FIG. 8 shows the guide-cap remover instrument according to one embodiment of the present invention, positioned over the guide cap of the system;
FIG. 9 is a closer view of the remover instrument according to one embodiment of the present invention, retrieving the guide cap;
FIG. 10 shows the remover instrument according to one embodiment of the present invention, holding the guide cap removed from the first monolithic percutaneous pedicle screw system, of FIG. 1, and positioned over a second guide cap of a second monolithic percutaneous pedicle screw system, like the system of FIG. 1, for forming a multi-system spinal construct in a patient;
FIG. 11 shows the remover instrument according to one embodiment of the present invention, holding first, second, and third guide caps removed from corresponding monolithic percutaneous pedicle screw systems, and positioned over a second guide cap of a fourth monolithic percutaneous pedicle screw system, like the system of FIG. 1, for forming a multi-system spinal construct in a patient;
FIG. 12 shows dispelling of guide caps from the cap remover instrument by action of a plunger sub-system of the instrument according to one embodiment of the present invention; and
FIG. 13 is a side cross-section of a proximal portion of a receiver assembly and a distal portion of an intrinsic extender both having a helical-flange threadform for receiving a helical-flanged setscrew.

### DETAILED DESCRIPTION

The present technology includes monolithic percutaneous-screw systems. The systems can be used in minimally invasive spinal surgeries, such as in the thoracic, thoracic-lumbar or lumbar regions.

Example surgeries include spinal surgeries for correcting or improving patients with adolescent idiopathic scoliosis, or AIS surgery.

Benefits of the present technology include obviating need for external extenders, saving manufacturing/product cost, storage space, shipping needs, and time and work in the procedure. Time is saved by none being needed to connect external extenders to a rod receiver, or any cap, for instance. There is also no chance to mis-assemble external extenders to the rod receiver or any cap.
extender instruments by a perc screw head having an extended the rod-slot height. Functionality of the systems is also benefited by geometries facilitating ease and safety in separation of select portions of the system after implantation and building of a rodded spinal-correction construct.

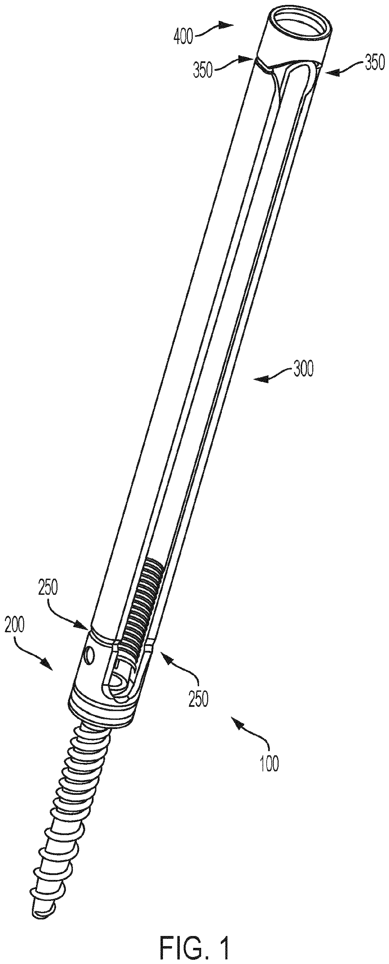

Turning now to the drawings, and more particularly to the first figure, FIG. 1 is a perspective view of a monolithic percutaneous pedicle screw system according to an example which does not fall within the scope of the claims as such but represents disclosure helping in understanding the present invention. The system is referenced by numeral 100 in the drawings.

The system 100 includes a distal receiver assembly 200 connected by an intermediate intrinsic extender component 300 to a proximal guide cap 400. The term intrinsic is used in connection with the nature of the extender 300 being connected monolithically, or unitarily, with the adjacent cap 400 and receiver 220.

The extender component 300 is connected to the receiver assembly 200 by a first, distal, breakoff section 250. And to the guided cap 400 by a second, proximal, breakoff section 350.

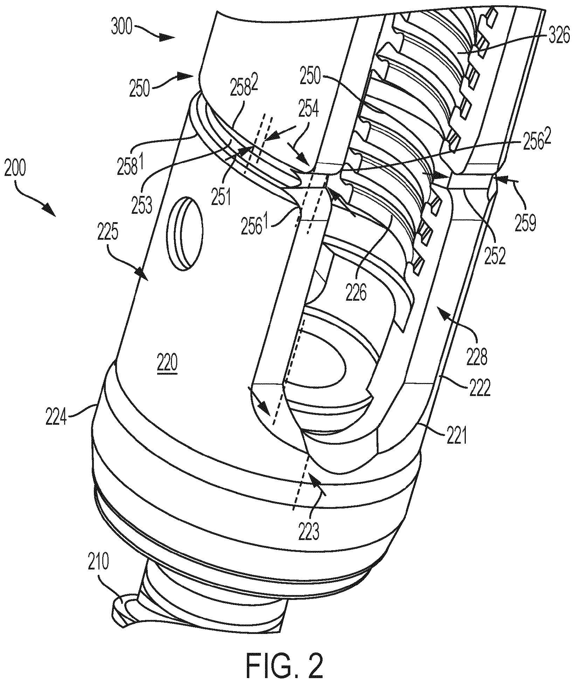

These components, sections, and a multiple-cap removing-and-holding instrument, are described further in turn with reference to FIGs. 2-13.

FIG. 2 is a perspective view of a proximal portion of the receiver assembly 200 and a distal portion of the intrinsic extender 300 of the system 100 of FIG. 1. The receiver assembly includes or is attached to a bonescrew 210, and a receiver 220 connected to the bonescrew 210. A head of the bonescrew 210 and head-receiving components of the receiver 220 are in various examples configured such that the head can be readily pushed into, or popped in, to the receiver 220 and the head-receiving components would hold the head and so the bonescrew in place against the receiver 220. In use of the system 100, the bonescrew 210 is anchored to a patient vertebra-to a pedical region thereof, for instance. The system 100 may in these cases include the term pedical, such as monolithic percutaneous pedical screw system.

The receiver assembly 200 can be configured in a uni-axial format such that the receiver 220 can be moved only along a single plane with respect to the bonescrew 210, or a multi-axial format such that the receiver 220 can be moved anywhere within a generally conical space with respect to the bonescrew 210. The receiver 220 and bonescrew 210 has, in an example, a fixed format, whereby the receiver 220 does not more with respect to the screw 210.

For multi- and uni-axial formats, a head of the bonescrew 210 extends into a distal cavity (not shown in detail) of the receiver 220, and the head is movable within the cavity.

The receiver 220 includes opposing receiver arms 222 extending from a receiver base 224. The arms 222 define a rod slot between them.

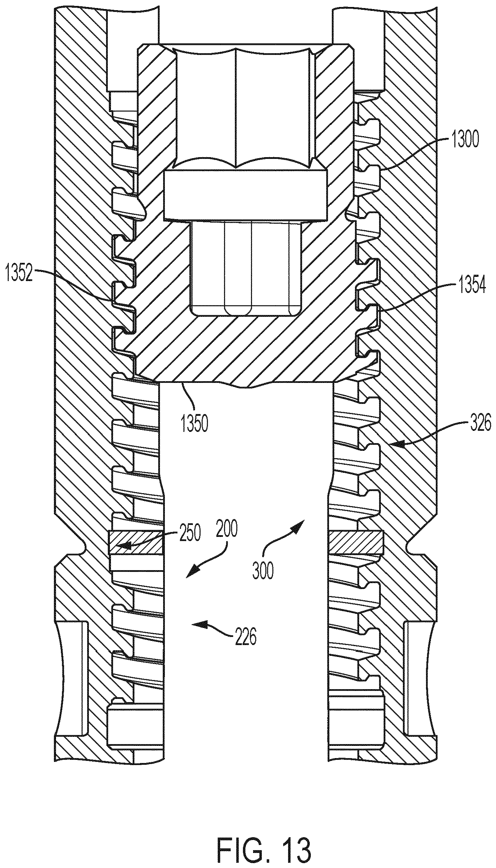

Each receiver arm 222 extends from a distal end to a proximal end, between side edges or walls 228, and between an outer wall and an inner wall having a threadform 226. The threadform in various examples has a helical-flange format, as described further in connection with FIG. 13.

The sidewalls 228 of the receiver arms 222 are in some examples recessed, setback, or offset, by a distance 223, from an outer diameter (whether a maximum OD) of the receiver base 224. Having the arms 222 extend radially out less than the base 224 gives the receiver 220 and so the system 100 a relatively lower profile, enabling improved visibility around the receiver 220, and lowering material cost and weight without compromising strength.

The setback arms 222 are connected to the base 224 by a curved transition 221. The gradual change promotes strength in the transition 221. And the gradual interface limits effects of edging, such as by lowering chances of a surgeon or assistant accidentally ripping a sterile glove in handling the receiver 220. The slight transition 221 also makes the area gentler on any adjacent patient tissue.

The first, distal, breakoff section 250 connects the receiver 200 to the intrinsic extender 300 monolithically. The section 250 is connected to the receiver 200 and the extender 300 in a unitary manner, for instance, verses being snapped, snug, fit, or otherwise connected to each other by an end user. The connection can include the receiver, breakoff section, and extender being formed together, or semi-permanently attached (such as by welding), in original manufacturing of the system 100, for example.

The breakoff section 250 can be configured in any of a variety of ways to be weaker for breaking. In some examples, the breakoff section 250 is weaker by (A) being thinner than a thickness (measured from inner wall to outer wall) of one or both of (i) arms 222 of the receiver 220 and (ii) the intrinsic extender 300, and/or (B) having a width (from end to end of the section 250) that is less than a width of the adjacent (i) arms 222 of the receiver 220 and/or (ii) the intrinsic extender 300. The section 250 can instead or also be configured for ready breaking based on its material, such as by including a material that is frangible or relatively brittle, relative to adjacent material of the arms 222 and/or extender 300.

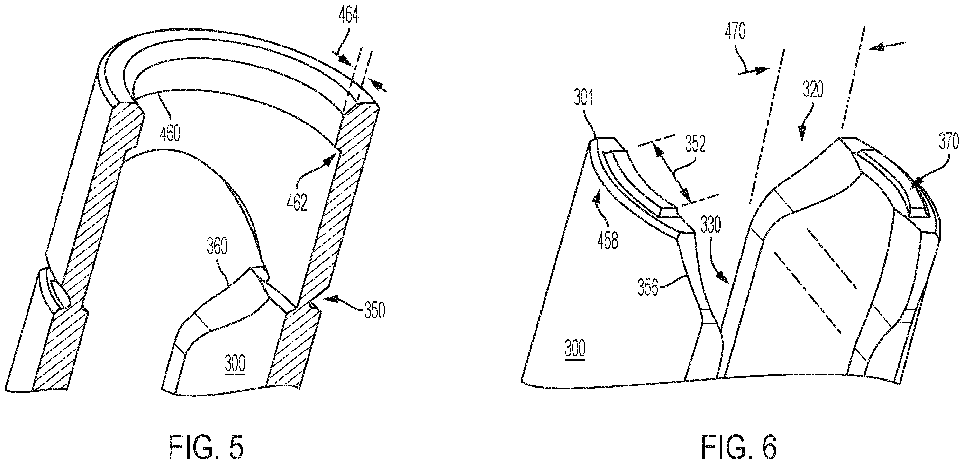

Portions of the breakoff section 250 are in various examples offset, recessed, or setback, in one or more portions. End walls 252 of the section 250 can be setback by a distance 254 from adjacent sidewalls 228 of the receiver 220, for instance. Less material at the breakoff section can have benefits including lower material cost and weight, without compromising strength of the section 250. Offsetting of the distal breakoff section 250 can also include an outer lateral surface 253 of the section 250 being spaced by a predetermined distance 251 from the adjacent outer wall 225 of the receiver 220. Benefits of the offsetting also lowers effects of edging, such as by lowering chances of the surgeon or assistant accidentally ripping or catching a sterile glove in handling the receiver 220 after the intrinsic extender 300 has been broken from the receiver 220 (reference FIG. 6). The broken breakoff section 250 may have some roughness, for instance, that can be better avoided with the section being setback from adjacent material in this way. The offsetting also makes the area gentler on any adjacent patient tissue after the break, as compared to the broken section 250 extending further or fully to adjacent surface(s) 225 of the arm 222, such as to the top of the arm wall 228 and to the outer wall of the receiver 220.

The receiver 220 can also have low-profile transition portions, for lowering weight, material cost, effect on adjacent tissue post-surgery, and especially the chance of ripping or catching a sterile surgical glove. Example transition portions include curved or beveled proximal side edges 258¹ of the receiver 220, and curved or beveled distal side edges 258² of the intrinsic receiver 300. The low-profile transition portions can also include beveled or curved side ends 256¹ of the receiver 220, and curved or beveled distal side ends 256² of the intrinsic receiver 300. Benefits of such receiver-extender include benefits analogous to any of the those provided above in connection with the breakoff-section offsetting.

The receiver 220 inner threadform 226 has a configuration corresponding to an inner threadform 326 of the intrinsic extender 300. The configuration includes sizing, shape, orientation, and positioning. The two threadforms 226, 326 are clocked to match each other, for instance, so that a setscrew 1350 (FIG. 13) can be readily and smoothly threaded from the extender threads to the receiver threads 226 for locking down the spinal rod (not shown) in the rod slot defined by the receiver arms 222.

In various examples, a height of the extender threadform, measured in a longitudinal, distal-to-proximal, direction, is greater than a height of the receiver threadforms. The extender-threadform height is in some cases greater than twice the receiver-threadform height.

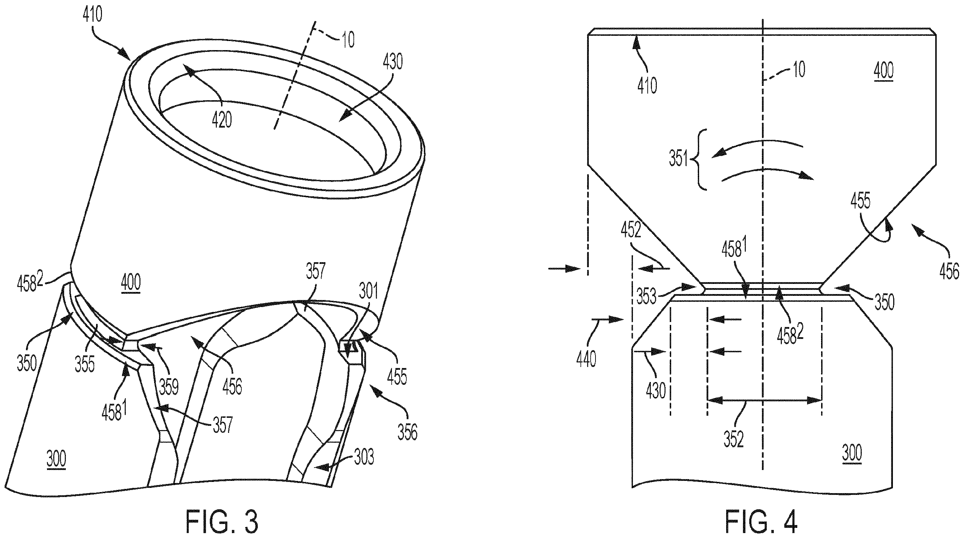

FIG. 3 is a perspective view of the guide cap 400. The cap 400 is in various examples generally cylindrical, and round in profile. Benefits of the cap being rounded can include the cap having no drastic edging at the proximal end, as compared to the higher edging formed by the proximal ends 301 of the extenders 300, which extend radially away from a centerline of the extender proximal end until a point at which the end surface terminates and the surface transitions (reference transition area 356 in FIG. 3, for instance), to extender side walls 303. The rounded proximal end of the cap 400 thereby lowers the chance of other objects, such as a surgeon's glove, implant or instrument catching on the cap 400. The round or circular shape of the cap 400, or at least proximal end thereof, also benefits the guiding function of the cap, better guiding instruments into and through the cap 400 and downstream channeling of the instrument along the longitudinal axis 10.

The guide cap 400 is connected monolithically by the second, proximal, breakoff section 350 to a proximal portion of the intrinsic extender 300 of the system 100 of FIG. 1. The section 350 is connected to the cap 400 and the extender 300 in a unitary manner, for instance, verses being snapped, snug fit, or otherwise connected to each other by an end user. The connection can include the cap, breakoff section, and extender being formed together, or semi-permanently attached (such as by welding), in original manufacturing of the system 100, for example.

The proximal breakoff section 350 can be configured in any of a variety of ways to be weaker for breaking. In some examples, the proximal breakoff section 350 is weaker by (A) having a thickness 359 that is thinner than a thickness (measured from inner wall to outer wall) of one or both of (i) adjacent walling of the guided cap 400 and (ii) adjacent walling of the intrinsic extender 300, or (B) having a width (from end to end of the section 350) that is less than a width of the adjacent (i) guide cap 300 and/or (ii) the intrinsic extender 300. The section 350 can instead or also be configured for ready breaking based on its material, such as by including a material that is frangible or relatively brittle (relative to adjacent material of the guide cap 400 and/or extender 300).

The second breakoff section 350 can be offset, recessed, or setback from lateral and side edges or walls of the intrinsic extender 300, in ways, and for analogous reasons, that the first breakoff section 250 can be setback in one or more portions, as described above in connection with FIG. 2.

In various examples, the second breakoff section 350 is configured to breakoff by application of a moment or force in a different manner than moment or force that snaps the first breakoff section 250. While the maneuver can involve application of a moment, force, or combination, the action is referred to for simplicity here as a moment, considered to include these unless explicitly described or claimed otherwise herein.

In some examples, the moment required to snap the first and second breakoff sections 250, 350 can be in opposite directions-e.g., along orthogonal or perpendicular planes. In one case, the second breakoff section 350 is configured to be broken along a sagittal plane, or in a sagittal direction, in the patient reference frame, and the first breakoff section 250 is configured to be broken off by medial-to-lateral moment, or in a medial-lateral direction.

Regarding the example breaking direction of the proximal breakoff section 350, which is in various implementations snapped before the distal breakoff section 250, reference is made to FIG. 4, in cases in which the system 100 is implanted such that the plane of the page having FIG. 4 would be along the patient's sagittal plane, then sagittal-directed moment would rock the top or proximal end of the cap 400 towards the left, or toward the right, along a curve. The sagittal motion is indicated generally by arrows 351 in the view.

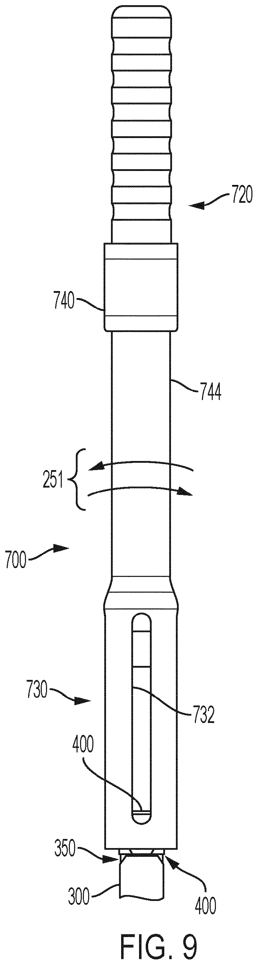

Regarding the example breaking direction of the distal breakoff section 350, which is in various implementations snapped after the proximal breakoff section 250, reference is made to FIG. 9. In cases in which the system 100 is implanted such that the plane of the page having FIG. 9 would be medial-lateral in the patient reference frame (i.e., the view looking at FIG. 9 would be looking sagittally, such as cranially or caudally (up or down the patient's spine), then application of a medial-lateral (or center, out) moment to the extender 300 would rock the proximal end of the extender 300 towards the left, or toward the right, along a curve. The medial-lateral motion is indicated generally by arrows 251 in the view.

Benefits of designing the system 100 so that the sections 250, 350 break most easily in response to moments in different directions, respectively, include better allowing a user to selectively break the system 100 at one of the sections without in that same motion breaking the system at the other section. The system 100 is in these examples designed for instance so that when a user applies a moment to the cap 400 along the sagittal plane, such as using the cap-removing tool 700, to readily snap the proximal breakoff section 350, the distal breakoff section 250 does not snap as well in the motion, the distal section 250 being designed to be stronger against sagittal-plane moment versus medial-lateral moment. The user can subsequently apply medial-lateral moment to the intrinsic extender 300, such as by an extender- or tab-breaking instrument (not shown), to snap the extender 300 at the distal section 250 from the receiver 220.

The system 100, including the breakoff sections 250, 350, can be configured in of various ways to promote the sections being more amenable to breaking in different respective directions. A primary example involves system 100 geometry, including size and shape. Various dimensions of the sections can be designed to promote breaking in response to predetermined moments. Width, length, and height are main examples. As an example, the relatively short length 352 of the proximal breakoff section 350 makes it easier to snap the cap 400 from the extender 300 at the section 350 by a moment applied to the cap along either direction 351 shown in FIG. 4, as compared to if the length 352 were longer. Regarding relative moment, or force, requirements for snapping between the two sections 250, 350, the proximal breakoff section 250 can have a greater length than the length 352 of the proximal section 350. A longer distal section 250 would resist snapping more in response to sagittal moment applied to the cap 400 and transmitted down to the section 250.

In contemplated examples, section surface shaping is configured to affect how the sections 250, 350 break, respectively. The proximal breakoff section 350 can have a curved (e.g., concave) end surface 353 (the short surface), making the section 350 easier to snap in response to moment along the directions 351 shown. In some examples, the surface 353 has at least one internal edge, or corner, such as by having a generally v-shape, versus having a fully curved concavity, and the concavity can be partially smooth leading generally radially inward to a generally v-shaped apex. Similarly, the distal breakoff section 250 can have a curved (e.g., concave) lateral surface 253 (the long surface), making the section 250 easier to snap in response to moment along the directions 251 (e.g., medial-lateral) shown in FIG. 9. A radially inner surface of the section 250, opposite the outer surface 253, can be curved for similar reasoning.

Along with breakoff-section material sizing and shaping being designed to make breaking easier in response to moments in select directions, sizing and shaping can be used to make breaking more difficult in response to moments in response to other directions. As an example, the end surface 252 of the distal breakoff section 250 is shown to be generally flat in FIG. 2, making it more difficult to snap the section 250 by moment applied to the system 100 along the sagittal plane (e.g., along the directions shown in FIG. 4). It is further contemplated that snap-resistance of the section 252 in response to moment in the sagittal plane can be increased by making the end surface 252 convex or being convex to some extent. Similar rationale can affect design of one or both lateral (radially inner and outer) surfaces 355, 357 (FIG. 3) of the proximal breakoff section 350. The surfaces 355, 357 can be designed to be flat or convex to some extent, for instance.

FIG. 4 is a side view of the parts shown in FIG. 3. Reference numeral 430 indicates a first example offset (e.g., sagittal-direction, or sagittal, offset), between a setback end surface 353 (measured from any point of the surface if not flat, such as at a radially inner most point) of the proximal breakoff section 350, and end or edge of the adjacent proximal end of the intrinsic extender 300.

FIGs. 3 and 4 also show the proximal end of the intrinsic extender 300 having a low-profile, or profile-lowering, transition portion 458¹, and the distal end of the guided cap 400 having a low-profile transition portion 458². The proximal end of the intrinsic extender 300 can also include side low-profile transition portions 356, which can include a slope 357. The slope 357 can be curved or arched, and/or at either end of the slope be beveled or curved.

In various examples, each of the side transition portions 356-e.g., the slopes 357 thereof-extends at an angle with respect to an adjacent side wall 303 of the extender of between about 30 and 60 degrees, such as between about 40 and 50 degrees, such as approximately 45 degrees, all as shown in FIGs. 3 and 4.

The receiver 220 can also have low-profile transition portions, for lowering weight, material cost, effect on adjacent tissue post-surgery, and especially the chance of ripping or catching a sterile surgical glove. Example transition portions include curved or beveled proximal side edges 258¹ of the intrinsic receiver 300, and curved or beveled distal side edges 258² of the guide cap 400.

The guide cap 400 can further include a proximal external transition area 410, such as a curved or beveled surface.

Benefits of the offsetting and transition portions lower effects of edges can include any of those described above regarding offsetting and transitioning between the receiver 220 and intrinsic extender 300. As there, regarding the first, distal, breakoff section 250, offsetting of the second breakoff 350 is especially beneficial after the cap 400 has been broken from the intrinsic extender 300, because the broken breakoff section 350 may have some roughness, for instance, that can be better avoided with the section 350 being setback from adjacent material in this way.

The offsetting also makes the portion gentler on any adjacent patient tissue after the break, as compared to the broken section 350 extending further or fully to proximal side surfaces of the intrinsic extender 300.

An outer diameter (OD) of the guide cap 400 may be greater than an OD of the intrinsic extender 300, as indicated by distance 452 in FIG. 3. Benefits of the larger relative OD include easier handling, including grasping, of the guide cap 400, manually or by tool, instrument, or machine. Benefits can also include snapping of the guide cap 400 from the extender 300 at the proximal breakoff 450 being easier.

FIGs. 3 and 4 show cutouts 456 of the guide cap 400, which may be curved or arched illustrated. Use herein of the term cutout does not limit the manner in which the associated geometry is formed. The related surfacing 456 does not have to be formed by cutting, or even machining, but can be. The cutouts 456 may be referenced herein, including in the claims, by other terms, such as transition portions 456.

In various examples, each cutout 456 has a surface 455 extending at an angle with respect to an adjacent side wall of the cap 400 and/or a side wall of the extender of between about 30 and 60 degrees, such as between about 40 and 50 degrees, such as approximately 45 degrees, all as shown in FIG. 4.

The cutouts 456 provide various benefits, including lowering weight, material for making, and cost, increasing in-procedure visibility, improved handling by a surgeon or assistance. The side low-profile transition portions 356 can also be viewed to create cutout sections instead of or along with the guide cap cutouts 456, to provide the same or similar benefits just mentioned in connection with, e.g., opposing, the cap cutouts 456, or increase the same benefits (even greater handleability, e.g.) when used with cap cutouts 456. The cutouts 456, the distal transition portions 356, or the two together can also provide robust clearance helpful in snapping the cap 400 from the extender 300 by moment along either direction 351 called out in FIG. 4.

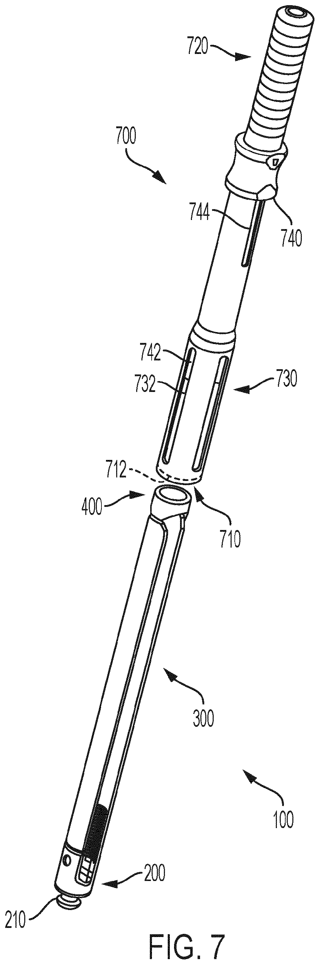

A central or longitudinal axis is referenced by numeral 10 in FIGs 3 and 4. Though not shown in every view, the axis represents the longitudinal axis for the system 100 as a whole, the components thereof 200, 300, 400, and for extrinsic parts and tools, such as the set screw 1350 (FIG. 13), instruments guided through the system channel, such as setscrew driver (not shown) or bone-filler device (not shown), and the multiple-cap removing-and-holding instrument 700 (FIG. 7).

The guide cap 400 include a proximal radially inner transition portion 420, in various examples. The transition portion 420 can be a curved or beveled surface. The proximal internal transition portion can provide analogous benefits to any of the benefits described above regarding other transition portions. The proximal internal transition portion 420 can also facilitate positioning (e.g., easier or otherwise better guiding) of media, such as instruments (e.g., a setscrew-driver or bone-filler tool), such as a setscrew driver (not shown), into and in the channel 430 formed by the cap 400 as well as into and in the aligned proximal channeling defined by the extender 300 and receiver 220.

The guide cap 400 also includes a retention feature 460 adjacent its proximal end in various examples. The retention features 460 can include an inward protrusion or lip 462. The retention feature 460 can be configured to engage a corresponding retention feature (not shown) of a driver, bone filler, or other instrument placed into the cap 400, by the instrument catching or attaching to the retention feature 460 of the cap for provisional retention of the retention feature of the instrument, and so of the instrument, in a desired position there with respect to the cap 400. The instrument retention feature has a geometry corresponding to geometry (e.g., size and shape) of the retention featured 460. The instrument may have a groove or other shaping machined or otherwise formed into an outer surface of the instrument, such as a distal or proximal surface, of the instrument, corresponding to the geometry of the cap retention features 460, for example.

FIG. 6 is a perspective view of the proximal portion of the intrinsic extender 300 after the guide cap has been broken off of the extender 300 at the proximal breakoff section 350. A section 370 of the breakoff section 350 remains at the proximal end 301 of the intrinsic extender 300.

As also shown in FIG. 6, the intrinsic extender 300 can include a transition surface 320 extending between a proximal end 301 of the extender 300 an intermediate inner wall 320 of the extender 300. The transition surface 320 can have various benefits, including by providing a higher relative strength distal of the surface 320, where a wall of the extender 300 (is thickest, or at least thicker than the extender wall is at the proximal-surface), while lowering lower material cost and weight. The transition surface 320 can also promote implant and instrument guidance, such as by guiding the setscrew 1350 (FIG 13), a setscrew driver (not shown), or bone-filler device (not shown), to stay along the central axis 10 (shown in FIGs. 4 and 5) from the channel of the cap 400 down through the channel of the extender 300 to the rod slot of the receiver 220.

In various examples, the extenders 300, due to the transitions 320, transition from having a proximal separation between them, matching generally an inner diameter of the guide cap 400, to a distal separation between them matching generally an inner diameter defined by inside walls of the receiver arms 222.

FIG. 7 shows the multiple-cap removing-and-holding instrument 700 adjacent the monolithic percutaneous pedicle screw system 100. The instrument is in various embodiments configured to remove and hold multiple guide caps 400, and so be referred to as a multiple-cap remover, a multi-cap-remover tool or instrument, or the like.

By being able to hold multiple caps at a time, the multiple-cap removing-and-holding instrument 700 saves movement and time in surgery, by obviating the need to empty the receiver after each cap 400 is removed, or to use a separate remover for each cap.

The multiple-cap removing-and-holding instrument 700 extends from a distal cap-receiving end 710 to a proximal handle end 720. The distal cap-receiving end 710 is configured (e.g., sized and shaped) to receive into an interior of the distal cap-receiving end 710 a cap 400 of the system 100.

The multiple-cap removing-and-holding instrument 700 includes a compartment 730 for holding one or more caps 400. The compartment 730 in various embodiments is a magazine, for holding multiple caps 400. The compartment 730 has side walls defining one or more windows or apertures 732, in various embodiments, providing visibility to its interior, allowing a user to see whether and, if any, how many caps 400 are in the compartment 730.

The multiple-cap removing-and-holding instrument 700 also includes in the compartment 730 an internal cap-pushing device, such as a plunger 742 movable with respect to the side walls of the compartment 730. The remover is configured such that a user can manipulate the remover to push the plunger 742 distally to push down on one or more caps stored in the compartment 730, to eject the caps from the remover 700. This makes room for the multiple-cap removing-and-holding instrument 700 to be used subsequently for capture more caps 400.

The multiple-cap removing-and-holding instrument 700 includes cap-retaining structure 712 adjacent its distal cap-receiving end 710. The cap-retaining structure 712 is configured (e.g., geometry (size, shape)) so that the distal cap-receiving end 710 of the multiple-cap removing-and-holding instrument 700 can be slid over a guide cap 400 readily, with relative ease. The cap-retaining structure 712 is further configured such that a guide cap 400 captured by the multiple-cap removing-and-holding instrument 700, upon passing the structure 712, cannot easily fall out of the distal cap-receiving end 710, such as by simple effect of gravity, or moving the multiple-cap removing-and-holding instrument 700 about the operating room, etc. The cap-retaining structure 712 is still further configured such that any captured guide caps 400 can be pushed proximally past the structure 712 by a cap-expelling sub-system of the remover 700, such as one including the plunger 742. In embodiments, the cap-retaining component 712 includes a lip 712 containing material, such as a plastic or rubber, that is softer than material of the chamber wall, which may include metal, for instance. The softer material gives as caps are pushed into or out of the compartment, but keeps them in when the only force on the cap/s is gravity or per usual, non-cap-expelling activity, by the user, so as not to fall out prematurely. This flexibility can instead or also be provided by slots in the distal end, so that the end includes fingers that can bend radially outward when pushed by a cap 400. This feature is also considered indicated schematically the reference numeral 712.

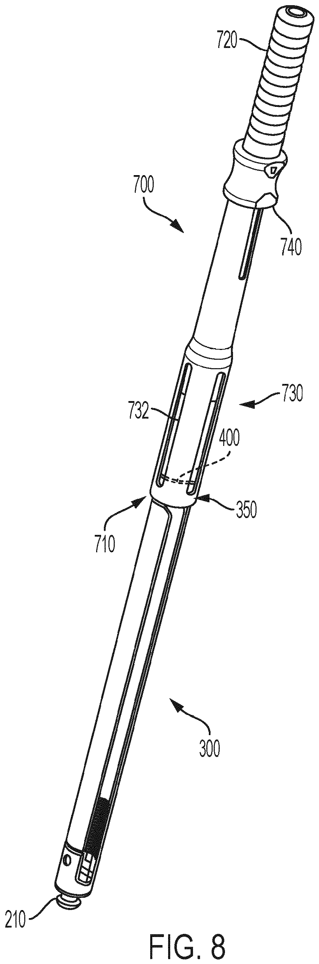

FIG. 8 shows the multiple-cap removing-and-holding instrument 700 having been positioned over the guide cap 400 of the system 100.

FIG. 9 is a closer view of the multiple-cap removing-and-holding instrument 700 retrieving the guide cap 400.

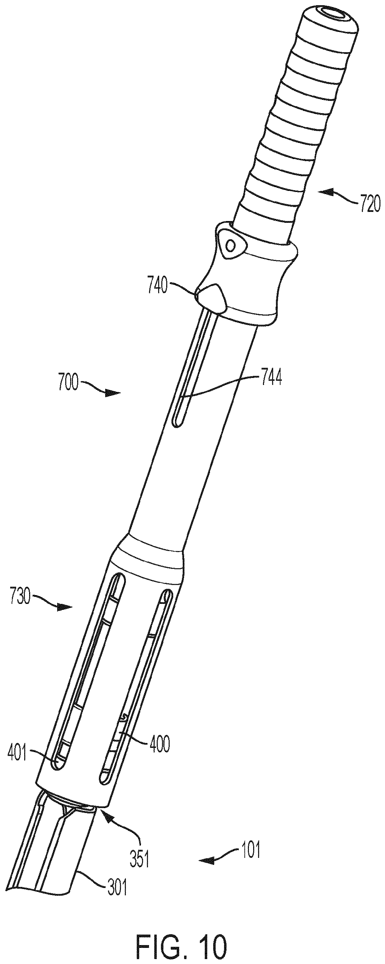

FIG. 10 shows the multiple-cap removing-and-holding instrument 700 holding the guide cap 400 removed from the first monolithic percutaneous pedicle screw system 100, of

FIG. 1, and positioned over a second guide cap 401 of a second monolithic percutaneous pedicle screw system 101, like the system of FIG. 1, for forming a multi-system spinal construct in a patient;

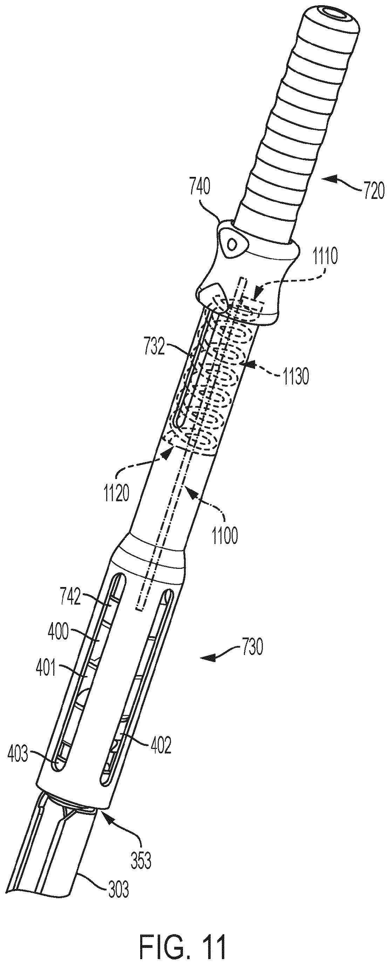

FIG. 11 shows the multiple-cap removing-and-holding instrument 700 holding first, second, and third guide caps 400, 401, 402 removed from corresponding monolithic percutaneous pedicle screw systems, and positioned over a fourth guide cap 403 of a second monolithic percutaneous pedicle screw system, like the system of FIG. 1, for forming a multi-system spinal construct in a patient (not shown).

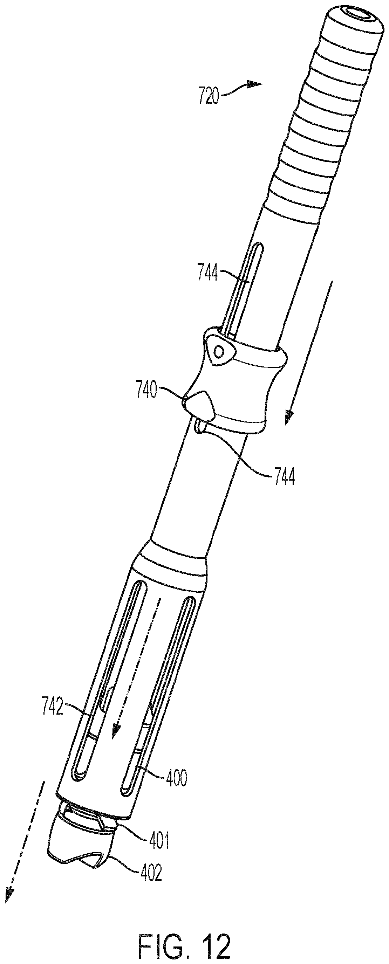

FIG. 12 shows dispelling of guide caps from the multiple-cap removing-and-holding instrument by action of the cap-pushing device of the multiple-cap removing-and-holding instrument 700, such as a device including the plunger 742.

In various embodiments, as shown in FIG. 11, the multiple-cap removing-and-holding instrument 700 has a spring foundation 1120, such as a flange, on which a spring 1130 sits or to which the spring is connected.

The spring 1130 is positioned within the body-intermediate the handle 720 and the compartment 730, e.g.-and in contact with the sliding component 740 to bias the sliding component proximally. The sliding component 740 in some cases includes a collar extending around the body. The multiple-cap removing-and-holding instrument 700 has an elongated actuator 1100 connecting the sliding component 740 to the plunger 742. In some cases, the spring 1130 at least partially surrounds the elongated actuator 1100 within the body.

In various embodiments, the body portion has a body wall defining a longitudinal slot 744 (FIG. 10). The sliding component 740 may have or be connected to a collar-actuator component 1110 connecting the sliding component 740 to the elongated actuator 1100, the collar-actuator component 1110 being slidably disposed in the slot 744.

Thus, the collar 740 is connected by the elongated actuator 1100 to the plunger 742, such that when the collar 740 is pushed distally by a user, the collar, connected to the actuator 1100, causes the elongated actuator 1100 to push the plunger 742 down, distally within the compartment 730. The plunger 742 is thus pushed down against any guide caps 400 positioned in the compartment 730, until the cap/s 400 are pushed out of the distal end of the instrument 700, beyond any distal cap-retaining structure 712, thereby being ejected or expelled from the instrument 700.

FIG. 13 is a side cross-section of a proximal portion of the receiver assembly 200 and a distal portion of the intrinsic extender 300 both having a helical-flange threadform for receiving a helical-flanged setscrew 1350, according to various examples which do not fall within the scope of the claims as such but represents disclosure helping in understanding the present invention. The threadforms 226, 326 in these examples include a proximal flange-receiving space 1300 for receiving a proximal flange 1354 of each thread section 1352. Benefits of the flanged threadform include reducing splay, or moving apart of the arms 222, of the receiver 220. This can be especially helpful when the receiver 220 or at least the arms 222 have a material that has more of a tendency to splay, but has other benefits, such as weight, cost, or machinability.

It should be understood that various aspects disclosed herein may be combined in combinations other than the combinations presented specifically in the description and the accompanying drawings. It should also be understood that, depending on the example, certain acts or events of any of the processes or methods described herein may be performed in other sequence, added, merged, or left out altogether (e.g., all described acts or events may not be necessary to carry out the techniques).

In addition, while certain aspects of this disclosure are described as being performed by a single module or unit for purposes of clarity, it should be understood that the techniques of this disclosure may be performed by a combination of units or modules associated with, for example, a medical device.

Unless defined specifically otherwise herein, all terms are to be given their broadest possible interpretation including meanings implied from the specification as well as meanings understood by those skilled in the art and/or as defined in dictionaries, treatises, etc. It must also be noted that, as used in the specification and the appended claims, the singular forms "a," "an," and "the" include plural referents unless otherwise specified, and that the terms "comprises" and/ or "comprising," when used in this specification, specify the presence of stated features, elements, and/or components, but do not preclude the presence or addition of one or more other features, steps, operations, elements, components, and/or groups thereof.

## Claims

1. A multiple-cap removing-and-holding instrument (700), for removing guide caps (400) for spinal-implant assemblies in surgery, the instrument (700) comprising:
a distal compartment portion comprising a chamber component (730), a distal-instrument end (710), and a plunger (742) movably disposed within the chamber component (730), wherein:
the chamber component (730) comprises a chamber wall having an inner surface and an outer surface and extending from a proximal-chamber end to the distal-instrument end (710) forming a distal opening, and comprises a cap-retaining component (712),
the distal opening is sized and shaped to allow the guide caps (400) to pass through the distal opening and into the chamber component (730) in use of the multiple-cap removing-and-holding instrument (700) , wherein the cap-retaining component (712) is configured so that the distal-instrument end (710) can be slid over the guide cap (400) to capture and retain the guide cap (400) within the chamber component (730), and
the chamber component (730) is sized and shaped to receive, one at a time, and hold simultaneously, multiple caps (400) removed from respective spinal-implant assemblies using the multiple-cap removing-and-holding instrument (700);
a proximal handle portion (720);
a body portion extending from a proximal-body end, connected to the handle portion (720), to a distal-body end connected to the distal compartment portion; and
a sliding component (740) connected movably to the body portion and connected to the plunger (742) such that the sliding component (740), when moved distally, in use of the multiple-cap removing-and-holding instrument (700), moves the plunger (742) distally to push the guide caps (400) captured in the chamber component (730) distally, beyond the cap-retaining component (712), and out of the chamber component (730) via the distal opening at the distal-instrument end (710) ;
**characterized in that** the multiple-cap removing-and-holding instrument (700) further comprises a spring (1130) positioned within the body portion in contact with the sliding component (740) to bias the sliding component proximally.

2. The multiple-cap removing-and-holding instrument (700) of claim 1, wherein the chamber component (730) has a cylindrical cross section.

3. The multiple-cap removing-and-holding instrument of claims 1 or 2, wherein:
the sliding component (740) comprises a collar extending around the body portion, and the multiple-cap removing-and-holding instrument (700) comprises an elongated actuator (1100) connecting the collar to the plunger (742); and
the spring (1130) at least partially surrounds the elongated actuator (1100) within the body portion.

4. The multiple-cap removing-and-holding instrument (700) of one of claims 1 to 3, wherein the sliding component (740) comprises a collar extending around the body portion, and the multiple-cap removing-and-holding instrument (700) comprises an elongated actuator (1100) connecting the collar to the plunger (742).

5. The multiple-cap removing-and-holding instrument (700) of claim 3 or 4, wherein the body portion comprises a body wall defining a longitudinal slot (744).

6. The multiple-cap removing-and-holding instrument (700) of claim 5, wherein the collar has or is connected to a collar-actuator component (1110) connecting the collar to the elongated actuator (1100), the collar-actuator component (1110) being slidably disposed in the slot (744).

7. The multiple-cap removing-and-holding instrument (700) of one of claims 1-6, wherein the cap-retaining component (712) extends radially inward from the inner surface of the chamber wall at or adjacent the distal-instrument end (710).

8. The multiple-cap removing-and-holding instrument (700) of claim 7, wherein the cap-retaining component (712) comprises a lip containing material softer than material of the chamber wall.

9. The multiple-cap removing-and-holding instrument (700) of claim 7 or 8, wherein the chamber wall defines at least one aperture (732) providing visibility into the chamber component (730), or
wherein the chamber wall defines at least three apertures (732), each providing visibility into the chamber component (730).

10. The multiple-cap removing-and-holding instrument (700) of one of claims 1-9, wherein the chamber component (730) is sized and shaped to hold simultaneously at least three caps (400, 401, 402) removed from respective spinal-implant assemblies using the multiple-cap removing-and-holding instrument (700).

11. The multiple-cap removing-and-holding instrument (700) of one of claims 1-9, wherein the chamber component (730) is sized and shaped to hold simultaneously at least four caps (400, 401, 402, 403) removed from respective spinal-implant assemblies using the multiple-cap removing-and-holding instrument (700).

12. The multiple-cap removing-and-holding instrument (700) of one of claims 1-11, wherein the chamber component (730) is sized and shaped to hold simultaneously at least five caps (400, 401, 402, 403) removed from respective spinal-implant assemblies using the multiple-cap removing-and-holding instrument (700).

## Patentansprüche

1. Instrument (700) zum Entfernen und Halten mehrerer Kappen zum Entfernen von Führungskappen (400) für Wirbelsäulenimplantatanordnungen während der Operation, wobei das Instrument (700) umfasst:
einen distalen Kompartmentabschnitt, der eine Kammerkomponente (730), ein distales Instrumentenende (710) und einen Kolben (742) umfasst, der beweglich innerhalb der Kammerkomponente (730) angeordnet ist, wobei:
die Kammerkomponente (730) eine Kammerwand mit einer Innenoberfläche und einer Außenoberfläche umfasst und sich von einem proximalen Kammerende zu dem distalen Instrumentenende (710) erstreckt, das eine distale Öffnung bildet, und eine Kappenhaltekomponente (712) umfasst,
die distale Öffnung bemessen und geformt ist, um es den Führungskappen (400) zu ermöglichen, durch die distale Öffnung und in die Kammerkomponente (730) bei Verwendung des Instruments (700) zum Entfernen und Halten mehrerer Kappen hindurchzugehen, wobei die Kappenhaltekomponente (712) so konfiguriert ist, dass das distale Instrumentenende (710) über die Führungskappe (400) geschoben werden kann, um die Führungskappe (400) innerhalb der Kammerkomponente (730) aufzunehmen und zu halten, und
die Kammerkomponente (730) so bemessen und geformt ist, um mehrere Kappen (400), die unter Verwendung des Instruments (700) zum Entfernen und Halten mehrerer Kappen von entsprechenden Wirbelsäulenimplantatanordnungen entfernt wurden, eine nach der anderen aufzunehmen und gleichzeitig zu halten;
einen proximalen Körperabschnitt (720);
einen Körperabschnitt, der sich von einem proximalen Körperende, das mit dem Griffabschnitt (720) verbunden ist, zu einem distalen Körperende erstreckt, das mit dem distalen Kammerabschnitt verbunden ist; und
eine Gleitkomponente (740), die beweglich mit dem Körperabschnitt verbunden ist und mit dem Kolben (742) verbunden ist, so dass die Gleitkomponente (740), wenn sie bei der Verwendung des Instruments (700) zum Entfernen und Halten mehrerer Kappen in distaler Richtung bewegt wird, den Kolben (742) in distaler Richtung bewegt, um die in der Kammerkomponente (730) gefangenen Führungskappen (400) in distaler Richtung über die Kappenhaltekomponente (712) hinaus und über die distale Öffnung am distalen Instrumentenende (710) aus der Kammerkomponente (730) herauszuschieben;
**dadurch gekennzeichnet, dass** das Instrument (700) zum Entfernen und Halten mehrerer Kappen ferner eine Feder (1130) umfasst, die innerhalb des Körperabschnitts in Kontakt mit der Gleitkomponente (740) positioniert ist, um die Gleitkomponente in proximaler Richtung vorzuspannen.

2. Instrument (700) zum Entfernen und Halten mehrerer Kappen nach Anspruch 1, wobei die Kammerkomponente (730) einen zylindrischen Querschnitt aufweist.

3. Instrument zum Entfernen und Halten mehrerer Kappen nach Anspruch 1 oder 2, wobei:
die Gleitkomponente (740) einen Kragen umfasst, der sich um den Körperabschnitt erstreckt, und das Instrument (700) zum Entfernen und Halten mehrerer Kappen einen länglichen Aktuator (1100) umfasst, der den Kragen mit dem Kolben (742) verbindet; und
die Feder (1130) den länglichen Aktuator (1100) innerhalb des Körperabschnitts zumindest teilweise umgibt.

4. Instrument (700) zum Entfernen und Halten mehrerer Kappen nach einem der Ansprüche 1 bis 3, wobei die Gleitkomponente (740) einen Kragen umfasst, der sich um den Körperabschnitt erstreckt, und das Instrument (700) zum Entfernen und Halten mehrerer Kappen einen länglichen Aktuator (1100) umfasst, der den Kragen mit dem Kolben (742) verbindet.

5. Instrument (700) zum Entfernen und Halten mehrerer Kappen nach einem der Ansprüche 3 oder 4, wobei der Körperabschnitt eine Körperwand umfasst, die einen Längsschlitz (744) definiert.

6. Instrument (700) zum Entfernen und Halten mehrerer Kappen nach Anspruch 5, wobei der Kragen eine Kragenaktuatorkomponente (1110) aufweist oder mit dieser verbunden ist, die den Kragen mit dem länglichen Aktuator (1100) verbindet, wobei die Kragenaktuatorkomponente (1110) verschiebbar in dem Schlitz (744) angeordnet ist.

7. Instrument (700) zum Entfernen und Halten mehrerer Kappen nach einem der Ansprüche 1 bis 6, wobei sich die Kappenhaltekomponente (712) von der Innenoberfläche der Kammerwand am oder in der Nähe des distalen Instrumentenendes (710) radial nach innen erstreckt.

8. Instrument (700) zum Entfernen und Halten mehrerer Kappen nach Anspruch 7, wobei die Kappenhaltekomponente (712) eine Lippe umfasst, die ein Material enthält, das weicher ist als das Material der Kammerwand.

9. Instrument (700) zum Entfernen und Halten mehrerer Kappen nach Anspruch 7 oder 8, wobei die Kammerwand mindestens eine Öffnung (732) definiert, die einen Einblick in die Kammerkomponente (730) ermöglicht, oder wobei die Kammerwand mindestens drei Öffnungen (732) definiert, die jeweils einen Einblick in die Kammerkomponente (730) ermöglichen.

10. Instrument (700) zum Entfernen und Halten mehrerer Kappen nach einem der Ansprüche 1 bis 9, wobei die Kammerkomponente (730) bemessen und geformt ist, um gleichzeitig mindestens drei Kappen (400, 401, 402) zu halten, die unter Verwendung des Instruments (700) zum Entfernen und Halten mehrerer Kappen von entsprechenden Wirbelsäulenimplantatanordnungen entfernt wurden.

11. Instrument (700) zum Entfernen und Halten mehrerer Kappen nach einem der Ansprüche 1 bis 9, wobei die Kammerkomponente (730) so bemessen und geformt ist, dass sie gleichzeitig mindestens vier Kappen (400, 401, 402, 403) hält, die unter Verwendung des Instruments (700) zum Entfernen und Halten mehrerer Kappen von entsprechenden Wirbelsäulenimplantatanordnungen entfernt wurden.

12. Instrument (700) zum Entfernen und Halten mehrerer Kappen nach einem der Ansprüche 1 bis 11, wobei die Kammerkomponente (730) bemessen und geformt ist, um gleichzeitig mindestens fünf Kappen (400, 401, 402, 403) zu halten, die unter Verwendung des Instruments (700) zum Entfernen und Halten mehrerer Kappen von entsprechenden Wirbelsäulenimplantatanordnungen entfernt wurden.

## Revendications

1. Instrument de retrait et de maintien de bouchons multiples (700) pour retirer des bouchons de guidage (400) des ensembles d'implants vertébraux en chirurgie, l'instrument (700) comprenant :
une partie de compartiment distale comprenant un composant de chambre (730), une extrémité d'instrument distale (710) et un piston (742) disposé de manière mobile à l'intérieur du composant de chambre (730), dans lequel :
le composant de chambre (730) comprend une paroi de chambre ayant une surface interne et une surface externe et s'étendant depuis une extrémité de chambre proximale jusqu'à l'extrémité d'instrument distale (710) formant une ouverture distale, et comprend un composant de retenue de bouchon (712),
l'ouverture distale est dimensionnée et façonnée pour permettre aux bouchons de guidage (400) de passer à travers l'ouverture distale et dans le composant de chambre (730) lors de l'utilisation de l'instrument de retrait et de maintien de bouchons multiples (700), dans lequel le composant de retenue de bouchon (712) est conçu de sorte que l'extrémité d'instrument distale (710) peut être coulissée sur le bouchon de guidage (400) pour capturer et retenir le bouchon de guidage (400) à l'intérieur du composant de chambre (730), et
le composant de chambre (730) est dimensionné et conçu pour recevoir, un à la fois, et maintenir simultanément, de multiples bouchons (400) retirés des ensembles d'implant vertébral respectifs à l'aide de l'instrument de retrait et de maintien de bouchons multiples (700) ;
une partie de poignée proximale (720) ;
une partie de corps s'étendant d'une extrémité de corps proximale, reliée à la partie de poignée (720), à une extrémité de corps distale reliée à la partie de compartiment distale ; et
un composant coulissant (740) relié de manière mobile à la partie de corps et relié au piston (742) de telle sorte que le composant coulissant (740), lorsqu'il est déplacé distalement, lors de l'utilisation de l'instrument de retrait et de maintien de bouchons multiples (700), déplace le piston (742) distalement pour pousser les bouchons de guidage (400) capturés dans le composant de chambre (730) distalement, au-delà du composant de retenue de bouchon (712), et hors du composant de chambre (730) par l'intermédiaire de l'ouverture distale au niveau de l'extrémité d'instrument distale (710) ;
**caractérisé en ce que** l'instrument de retrait et de maintien de bouchons multiples (700) comprend en outre un ressort (1130) positionné à l'intérieur de la partie de corps en contact avec le composant coulissant (740) pour solliciter le composant coulissant de manière proximale.

2. Instrument de retrait et de maintien de bouchons multiples (700) selon la revendication 1, dans lequel le composant de chambre (730) a une section transversale cylindrique.

3. Instrument de retrait et de maintien de bouchons multiples selon les revendications 1 ou 2, dans lequel :
le composant coulissant (740) comprend un collier s'étendant autour de la partie de corps, et l'instrument de retrait et de maintien de bouchons multiples (700) comprend un actionneur allongé (1100) reliant le collier au piston (742) ; et
le ressort (1130) entoure au moins partiellement l'actionneur allongé (1100) à l'intérieur de la partie de corps.

4. Instrument de retrait et de maintien de bouchons multiples (700) selon l'une des revendications 1 à 3, dans lequel le composant coulissant (740) comprend un collier s'étendant autour de la partie de corps, et l'instrument de retrait et de maintien de bouchons multiples (700) comprend un actionneur allongé (1100) reliant le collier au piston (742).

5. Instrument de retrait et de maintien de bouchons multiples (700) selon la revendication 3 ou 4, dans lequel la partie de corps comprend une paroi de corps définissant une fente longitudinale (744).

6. Instrument de retrait et de maintien de bouchons multiples (700) selon la revendication 5, dans lequel le collier a ou est relié à un composant actionneur de collier (1110) reliant le collier à l'actionneur allongé (1100), le composant actionneur de collier (1110) étant disposé de manière coulissante dans la fente (744).

7. Instrument de retrait et de maintien de bouchons multiples (700) selon l'une des revendications 1-6, dans lequel le composant de retenue de bouchon (712) s'étend radialement vers l'intérieur à partir de la surface interne de la paroi de chambre au niveau ou adjacente à l'extrémité d'instrument distale (710).

8. Instrument de retrait et de maintien de bouchons multiples (700) selon la revendication 7, dans lequel le composant de retenue de bouchon (712) comprend une lèvre contenant un matériau plus mou que le matériau de la paroi de chambre.

9. Instrument de retrait et de maintien de bouchons multiples (700) selon la revendication 7 ou 8, dans lequel la paroi de chambre définit au moins une ouverture (732) fournissant une visibilité dans le composant de chambre (730), ou
dans lequel la paroi de chambre définit au moins trois ouvertures (732), chacune fournissant une visibilité dans le composant de chambre (730).

10. Instrument de retrait et de maintien de bouchons multiples (700) selon l'une des revendications 1-9, dans lequel le composant de chambre (730) est dimensionné et conçu pour maintenir simultanément au moins trois bouchons (400, 401, 402) retirés des ensembles d'implant vertébral respectifs à l'aide de l'instrument de retrait et de maintien de bouchons multiples (700).

11. Instrument de retrait et de maintien de bouchons multiples (700) selon l'une des revendications 1-9, dans lequel le composant de chambre (730) est dimensionné et conçu pour maintenir simultanément au moins quatre bouchons (400, 401,402, 403) retirés des ensembles d'implant vertébral respectifs à l'aide de l'instrument de retrait et de maintien de bouchons multiples (700).

12. Instrument de retrait et de maintien de bouchons multiples (700) selon l'une des revendications 1-11, dans lequel le composant de chambre (730) est dimensionné et conçu pour maintenir simultanément au moins cinq bouchons (400, 401,402, 403) retirés des ensembles d'implant vertébral respectifs à l'aide de l'instrument de retrait et de maintien de bouchons multiples (700).
